(19)

Europäisches
Patentamt
European
Patent Office
Office européen
des brevets

(11) **EP 2 362 225 A1**

(12) **EUROPEAN PATENT APPLICATION**

(43) Date of publication:
**31.08.2011 Bulletin 2011/35**

(51) Int Cl.:
*G01N 33/68* [(2006.01)]

(21) Application number: **10075087.6**

(22) Date of filing: **25.02.2010**

<table>
<tr><td>

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicant: **Charité Universitätsmedizin Berlin 10117 Berlin (DE)**

</td><td>

(72) Inventor: **The designation of the inventor has not yet been filed**

(74) Representative: **Gulde Hengelhaupt Ziebig & Schneider Patentanwälte - Rechtsanwälte Wallstrasse 58/59 10179 Berlin (DE)**

</td></tr>
</table>

(54) **Method for indentification of proteasome generated spliced peptides**

(57) The present invention is directed to a method for identifying spliced peptides of a parent peptide present in a biological sample, comprising the steps: a) providing an amino acid sequence of a parent peptide; b) determining all combinatorial amino acid members that can be generated by combination of two arbitrary fragments derivable from the amino acid sequence of the parent peptide and which thereby represent a potential spliced peptide of the parent peptide, with the proviso that each combinatorial amino acid member comprises a sequence of more than one amino acid; c) preparing a data set comprising determined combinatorial amino acid members, wherein each combinatorial amino acid member of the data set is assigned to its respective mass-to-charge ratios (m/z values); d) determining the mass-to-charge ratios (m/z values) for at least one peptide or peptide fragment present in or derived from a biological sample; e) comparing the mass-to-charge ratio (m/z value) of the at least one peptide or peptide fragment with the mass-to-charge ratios (m/z value) comprised in the data set; and f), if the mass-to-charge ratio (m/z value) of the at least one peptide or peptide fragment and a mass-to-charge ratio (m/z value) of a combinatorial amino acid member of the data set is identical or its deviation does not exceed a given threshold value, identifying the at least one peptide or peptide fragment as spliced peptide of the parent peptide and optionally assigning to the at least one peptide or peptide fragment the corresponding amino acid sequence of the respective combinatorial amino acid member of the data set.

Fig. 1

EP 2 362 225 A1

**Description**

The 20S proteasome particle with its active site β-subunits β1, β2 and β5 is a N-terminal nucleophilic hydrolase. It is the central catalytic unit of the ubiquitin proteasome system (UPS) and the catalytic core of the 26S proteasome that is built by the association of the two 19S regulator complexes with the catalytic 20S proteasome particle core. As part of the 26S proteasome the 20S proteasome particle degrades poly-ubiquitinated proteins to peptides of 3 to 20 residues in length (Schwartz AL, Ciechanover A (2009) Targeting proteins for destruction by the ubiquitin system: implications for human pathobiology. Annu Rev Pharmacol Toxicol 49: 73-96). IFN-γ induces the synthesis of alternative catalytic subunits and the concomitant formation of immunoproteasomes previously connected the UPS with the generation of virus or tumor derived antigenic peptides bound by MHC class I molecules and presented at the cell surface to CD8+ cytotoxic T lymphocytes (CTL) for immune recognition (Kloetzel PM (2001) Antigen processing by the proteasome. Nat Rev Mol Cell Biol 2: 179-187).

Until recently it appeared to be a canonical rule that peptides generated by the 20S proteasome particle are peptide fragments with a sequence identical to a linear contiguous sequence part present in the unprocessed parental protein. However, using patient derived CTLs and breaking this rule, two new epitope peptides were recently identified which were composed of two different peptide fragments of a parental protein and whose sequence was not identical to a linear sequence part present in the parent protein. These peptides were the result of a new mechanism called peptide-splicing and it was shown that proteasomes generated these spliced epitope peptides both *in vivo* and *in vitro* (Vigneron N, Stroobant V, Chapiro J, Ooms A, Degiovanni G, et al. (2004) An antigenic peptide produced by peptide splicing in the proteasome. Science 304: 587-590; and Warren EH, Vigneron NJ, Gavin MA, Coulie PG, Stroobant V, et al. (2006) An antigen produced by splicing of noncontiguous peptides in the reverse order. Science 313: 1444-1447). Proteasome catalyzed peptide splicing was proposed to be a transpeptidation reaction whereby the acylester intermediate is stabilized at the active site formed by the N-terminal threonine of the catalytic subunits for a time span that is sufficient to allow the N-termini of released peptide fragments to make a nucleophilic attack on the ester bond of the acyl-enzyme intermediate thereby forming a new peptide bond and producing the spliced peptides (Borissenko L, Groll M (2007) Diversity of proteasomal missions: fine tuning of the immune response. Biol Chem 388: 947-955). Furthermore, it was shown that two non-contiguous peptides can also be fused by splicing in a reversed order.

Unfortunately, so far the identification of proteasome spliced peptides remained entirely dependent on the accidental availability of respective cytotoxic T-cells, thus preventing a systematic investigation of this new proteasome process.

It is an object of the present invention to provide a solution to this limitation.

According to an aspect of the present invention a method is provided for identifying spliced peptides of a given parent peptide, in particular for identifying proteasome spliced peptides, in a biological sample. The method of the invention comprises the steps:

a) providing an amino acid sequence of a parent peptide;

b) determining all combinatorial amino acid members that can be generated by combination of two arbitrary fragments derivable from the amino acid sequence of the parent peptide and which thereby represent a potential spliced peptide of the parent peptide, with the proviso that each combinatorial amino acid member comprises a sequence of more than one amino acid;

c) preparing a data set comprising all or less then all determined combinatorial amino acid members, wherein each combinatorial amino acid member of the data set is assigned to its respective mass-to-charge ratios (m/z values);

d) determining a mass-to-charge ratio (m/z value) for at least one peptide or peptide fragment present in or derived from a biological sample;

e) comparing the mass-to-charge ratio (m/z value) of the at least one peptide or peptide fragment derived from a biological sample with the mass-to-charge ratios (m/z value) comprised in the data set; and

f) if the mass-to-charge ratio (m/z value) of the at least one peptide or peptide fragment derived from a biological sample and a mass-to-charge ratio (m/z value) of a combinatorial amino acid member of the data set is identical or its deviation does not exceed a given threshold value, identifying the at least one peptide or peptide fragment of the biological sample as spliced peptide of the parent peptide and optionally assigning to the at least one peptide or peptide fragment of the biological sample the corresponding amino acid sequence of the respective combinatorial amino acid member of the data set.

It has been found that it is possible to specifically determine spliced peptides, in particular to determine proteasome spliced peptides, of a given parent peptide of interest with a known amino acid sequence by creation of a data set containing all theoretically possible spliced peptides derivable from said parent peptide, by calculation of the corresponding mass-to-charge ratios (m/z values) and by subsequent comparison of the m/z-values of at least one peptide or peptide fragment derived from a given sample with the m/z-values of the data set.

This method allows for the first time the identification of so far unknown spliced peptides in a biological sample and, thus, allows for identification of so far undiscovered epitopes that may be involved in pathogenic or physiological processes. The method of the invention renders the detection of spliced peptides independent from the need of the presence of respective CTLs. Knowledge of such novel epitopes may lead to novel targets for the development of novel treatments against diseases like cancer, autoimmune diseases or the like.

The method of the invention is directed to the identification of spliced peptides in a biological sample, wherein the spliced peptides to be identified are spliced peptides of a given parent peptide with known amino acid sequence.

For the purpose of the present invention, the word "peptide" refers to any organic compound that comprises at least two amino acids which are covalently connected via a peptide bond. Within the peptide, the single amino acids are connected to each other in a defined manner in order to give a chain with a particular amino acid sequence. The term "peptide" encompasses amino acid sequences with a chain length of at least two amino acids, oligopeptides, polypeptides, as well as whole proteins or fragments thereof.

A **spliced peptide** is a peptide that consists of an amino acid sequence wherein one part of the sequence of the spliced peptide is identical to a first part of the linear amino acid sequence of a parent peptide and the remaining part of the sequence of the spliced peptide is identical to a second part of the linear amino acid sequence of said parent protein, with the proviso that the complete linear amino acid sequence of the spliced peptide does not find an identical contiguous counterpart in the linear amino acid sequence of the parent peptide.

A spliced peptide can be generated by cutting the parent peptide into fragments and by rearranging two of these fragments in an order that differs from the original order in the uncut parent peptide. Such rearrangement e.g. encompasses deletion of sequences originally present between the two fragments or reversing the order of the two fragments or combinations thereof.

A spliced peptide may exhibit a cis configuration and as such may be generated exclusively and solely by combination of two distinct fragments derivable from only one single molecule of the parent peptide. In other words, the spliced peptide in *cis* configuration cannot be composed of overlapping fragments of the parent peptide. Thus, a spliced peptide in cis configuration can never exhibit a sequence length identical or longer than the sequence length of the parent peptide.

A spliced peptide may exhibit a *trans* configuration and as such may be generated exclusively and solely by combination of two distinct fragments derivable only from two distinct molecules of the amino acid sequence of the parent peptide. In other words, the spliced peptide in *trans* configuration can only be composed of two fragments of the parent peptide that overlap at least partially. Thus, spliced peptides in *trans* configuration encompass solely spliced peptides that cannot be defined as being in cis configuration. Spliced peptides in *trans* configuration may exhibit (but do not have to exhibit) a sequence length identical or longer than the sequence length of the parent peptide.

The term **"parent peptide"** denotes a peptide with a known amino acid sequence for which the spliced peptides thereof shall be determined.

A **biological sample** as referred to in the present application is any sample comprising at least one peptide or peptide fragment. The biological sample may comprise more than one peptide or peptide fragment. The biological probe may be a complex sample. The biological sample may be derived from a biological source and/or from any non-biological source. Preferably, the biological sample comprises the at least one peptide or peptide fragment in solution and/or in suspension.

The method of the invention can be used to identify proteasome spliced peptides, i.e. spliced peptides produced by proteasomal processing of a parent peptide. **Proteasomes** are large protein complexes present in cells of all eukaryotes and archaea, as well as in some bacteria. In eukaryotes, they are located in the nucleus and the cytoplasm. The proteasome subcomponents are usually referred to by their Svedberg sedimentation coefficient (denoted S). The most common form of the proteasome is known as the 26S proteasome, which is about 2000 kilodaltons (kDa) in molecular mass and contains one catalytic 20S proteasome particle structure and two 19S regulatory caps. The 20S proteasome particle core is hollow and provides an enclosed cavity in which proteins are degraded; openings at the two ends of the core allow the target protein to enter. Each end of the 20S proteasome particle core associates with a 19S regulatory subunit that contains multiple ATPase active sites and ubiquitin binding sites. It is this structure that recognizes polyubiquitinated proteins and transfers them to the catalytic 20S proteasome particle core. An alternative form of regulatory subunit, called the 11S particle, can associate with the 20S proteasome particle core in essentially the same manner as the 19S particle; the 11S may play a role in degradation of foreign peptides such as those produced after infection by a virus.

**20S proteasome particle:** The number and diversity of subunits contained in the 20S proteasome particle depends on the organism. The number of distinct and specialized subunits is larger in multicellular than unicellular organisms and larger in eukaryotes than in prokaryotes. All 20S proteasomal particles consist of four stacked heptameric ring structures that are themselves composed of two different types of subunits; $\alpha$ subunits are structural in nature, whereas $\beta$ subunits are predominantly catalytic. The outer two rings in the stack consist of seven a subunits each, which serve as docking domains for the regulatory particles. The alpha subunits N-termini form a gate that blocks unregulated access of substrates to the interior cavity. The inner two rings each consist of seven $\beta$ subunits and contain the protease active sites that perform the proteolysis reactions. The size of the proteasome is relatively conserved and is about 150 angstroms (Å) by 115 Å. The interior chamber is at most 53 Å wide, though the entrance can be as narrow as 13 Å, suggesting that substrate proteins must be at least partially unfolded to enter. In archaea, such as *Thermoplasma acidophilum,* all the $\alpha$ and all the $\beta$ subunits are identical, while in 20S proteasome particles of eukaryotic origin, such as those in yeast, contain seven distinct types of each subunit. In mammals, the $\beta1$, $\beta2$, and $\beta5$ subunits are catalytic; although they share a common mechanism, they have three distinct substrate specificities considered chymotrypsin-like, trypsin-like, and caspase-like, respectively. Alternative $\beta$ forms denoted $\beta1i$, $\beta2i$, and $\beta5i$ can be expressed in cells in response to exposure to pro-inflammatory signals such as cytokines, in particular, interferon gamma. The proteasome assembled with these alternative subunits (also called **immunosubunits**) is known as the **immunoproteasome,** whose cleavage-site preference is altered relative to the **standard proteasome** (i.e. proteasome with $\beta1$, $\beta2$, and $\beta5$ subunits), see also Nandi D et al. (2006). "The ubiquitin-proteasome system". J Biosci 31 (1): 137-55; Kloss A, Meiners S, Ludwig A, Dahlmann B (2009) Multiple cardiac proteasome subtypes differ in their susceptibility to proteasome inhibitors. Cardiovasc Res; Mishto M, Santoro A, Bellavista E, Sessions R, Textoris-Taube K, et al. (2006) A structural model of 20S immunoproteasomes: effect of LMP2 codon 60 polymorphism on expression, activity, intracellular localisation and insight into the regulatory mechanisms. Biol Chem 387: 417-429.

**Step a):**

The method of the invention comprises the step of providing an amino acid sequence of a parent peptide. Said amino acid sequence will form basis for generation of the amino acid sequences of potential spliced peptides of the parent peptide. Thus, provision of the amino acid sequence defines the parent peptide and determines the scope and nature of spliced peptides that can be identified with the method of the invention. In a preferred embodiment of the method of the invention, the amino acid sequences of more than one parent peptide can be provided. This allows the identification of spliced peptides of more than one parent peptide. In a particular preferred embodiment, the amino acid sequences of a proteome or of all peptides with kown amino acid sequence of a proteome are provided in order to identify spliced peptides of members of the proteome. The **proteome** is the entire set of proteins expressed by a genome, cell, tissue or organism. More specifically, it is the set of expressed proteins in a given type of cells or an organism at a given time under defined conditions.

**Step b):**

When the amino acid sequence of one or more parent peptides has been provided, this sequence information forms basis for subsequent steps. Based on the sequence information provided, all combinatorial amino acid members (also called PSP, PSP stands for possible spliced peptide) are determined that can be generated by combination of two arbitrary fragments derivable from the linear amino acid sequence of the parent peptide, with the proviso that each combinatorial amino acid member comprises a sequence of more than one amino acid.

Each **combinatorial amino acid member** (PSP) represents a specific, unique amino acid sequence of a spliced peptide that is theoretically derivable from the amino acid sequence of the given parent peptide.

The combinatorial amino acid members (PSPs) are generated by calculating all combinations of two arbitrary fragments derivable from the amino acid sequence of the given parent peptide which result in an amino acid sequence not being idential over the whole sequence length with a linear, contiguous amino acid sequence of the parent peptide. Thus, a set of combinatorial amino acid members is generated that represents all theoretically possible spliced peptides derivable from the amino acid sequence of the parent peptide provided in step a).

Calculation of all theoretically possible combinatorial amino acid members (PSPs) can be performed as described below:

The digestion of the substrate of length L containing amino acids $a_i$ , mit $i=1..L$ results in $S_{PCP} = \frac{1}{2} (L - L_{ext} + 1)(L - L_{ext} + 2)$ possible cleavage products (PCP) each of which can be denoted as $PCP_{ij}$, wherein the PCP starts at the position $i$, wobei $i$ ausgewählt sein kann aus $i=1...L-L_{ext}+1$ (C-terminus), and ends at the position j, wobei j ausgewählt sein kann aus $j=i+L_{ext}-1...L$ (N-terminus). $L_{ext}$ describes the minimal length of a PCP that can be used to produce a PSP. Any two $PCP_{ij}$ and $PCP_{kl}$, may be spliced into $PSP_{i\bar{j}k\bar{l}}$. For the total amount of generated

products $S_{all}$, including PCP and PSP, we have i=1...L-L$_{ext}$+1, j=i+L$_{ext}$-1...L, k=1...L-L$_{ext}$+1, l=k+L$_{ext}$-1...L and we can calculate

$$S_{all} = \sum_{i=1}^{L-L_{ext}+1} \sum_{j=i+L_{ext}-1}^{L} \sum_{k=1}^{L-L_{ext}+1} \sum_{l=k+L_{ext}-1}^{L} 1 .$$

Note that

$$S_{all} = \frac{1}{4}(L - L_{ext} + 1)^2 (L - L_{ext} + 2)^2 = S_{PCP}^2 .$$

Combinatorial amino acid members (PSP) can be classified into the two main groups, i.e. cis configuration (PSP$_{cis}$) and *trans* configuration (PSP$_{trans}$), whereby cis splicing occurs in the same order as in the substrate (PSP$_{cis,normal}$, where i+L$_{ext}$≤j+1≤k+1≤l-L$_{ext}$) or in reverse order (PSP$_{cis,reverse}$, k+L$_{ext}$≤l+1≤i≤j-L$_{ext}$+1). The total number of all PSP is then $S_{PSP}$ = ¼-(L - L$_{ext}$ + 3)(L - L$_{ext}$ + 2)(L - L$_{ext}$ + 1)(L -L$_{ext}$) . The number of *trans* PSP can be calculated as PSP$_{trans}$=S$_{PSP}$-PSP$_{cis,normal}$- PSP$_{cis,reverse}$. In Table 1 the conditions for each product and their total amount are summarized.

**Table 1** *Computation of cleavage and splicing products. Described are the conditions to compute all products of a specific type (PCP, cis-normal PSP and -reverse PSP). The indices i, j, k and I are the amino acid positions of the product, e.g. PSP$_{i-j,k-l}$, L is the length of the substrate, L$_{ext}$ is the minimal length of distance between the two PCP that are ligated.*

| products | conditions | total amount |
|---|---|---|
| all fragments | i=1...L-L$_{ext}$+1,<br>j=i+L$_{ext}$-1... L,<br>k=1...L-L$_{ext}$+1,<br>1=k+L$_{ext}$-1...L | $\frac{1}{4}(L - L_{ext} + 1)^2 (L - L_{ext} + 2)^2$ |
| PCP | i=1...L-L$_{ext}$+ 1 | $\frac{1}{2}(L - L_{ext} + 1)(L - L_{ext} + 2)$ |
| cis - normal | i=1...L-2L$_{ext}$<br>j=i+L$_{ext}$-1...L-L$_{ext}$-1<br>k=j+2...L-L$_{ext}$+ 1<br>1=k+L$_{ext}$-1...L | $\frac{1}{24}(L - 2L_{ext} + 1)(L - 2L_{ext})(L + 3 - 2L_{ext})(L + 2 - 2L_{ext})$ |
| cis - reverse | k=1...L-2L$_{ext}$+1<br>1=k+L$_{ext}$-1...L-L$_{ext}$<br>i=1+1...L-L$_{ext}$<br>j=i+L$_{ext}$-1...L | $\frac{1}{24}(L - 2L_{ext} + 1)(L + 4 - 2L_{ext})(L + 3 - 2L_{ext})(L + 2 - 2L_{ext})$ |

**Step c):**

Based on the calculated combinatorial amino acid members, a data set is prepared which comprises all or part of the determined combinatorial amino acid members, i.e. all or part of the total number of potential spliced peptides of the parent peptide. When said data set is prepared, each combinatorial amino acid member of the data set is assigned to its respective mass-to-charge ratio (m/z value). The respective m/z values are calculated based on the sequence information for each combinatorial amino acid member of the data set. Preferably, the mass-to-charge ratio (m/z value) of an amino acid sequence of a determined combinatorial amino acid member is calculated based on its molecular weight and for charge states z = 1, 2 and/or 3. The person skilled in the art is well aware of how such m/z values can be calculated.

The data set comprising determined combinatorial amino acid members may comprise all or less combinatorial amino acid members that are theoretically derivable from the parent peptide and/or that have been determined in step b). Preferably, in the method of the invention the total number of combinatorial amino acid members present in the data set is reduced to less than the total number of all combinatorial amino acid members determined in step b). Said reduction can be performed in one of the following ways or in combinations of more than one of the following ways:

- the determined combinatorial amino acid members of the data set of step c) consist of combinatorial amino acid members which can be generated solely by combination of two arbitrary fragments derivable from one single molecule of the amino acid sequence of the parent peptide, i.e. the data set is restricted to combinatorial amino acid members with cis configuration;

- the determined combinatorial amino acid members of the data set of step c) consist of combinatorial amino acid members which can be generated solely by combination of two arbitrary fragments derivable from two distinct molecules of the amino acid sequence of the parent peptide, i.e. the data set is reduced to contain solely combinatorial amino acid members with *trans* configuration;

- the combinatorial amino acid members of the data set of step c) are reduced to combinatorial amino acid members with an amino acid sequence length that is usually presented or presentable by a MHC class I molecule on the surface of a cell, preferably with an amino acid sequence length of 3 to 20 amino acids, more preferably of 5 to 15 amino acids, even more preferably of 7 to 12 amino acids, most preferably 8 to 10 amino acids;

- the combinatorial amino acid members in the data set of step c) are reduced to combinatorial amino acid members with a pre-selected affinity to a MHC class I molecule. Said pre-selected affinity may be selected for a given particular MHC class I molecule, e.g. of a specific haplotype, or for any MHC class I molecule, irrespective of the haplotype. Such a pre-selected affinity can be expressed as a threshold value of a theoretical and/or arbitrary value that is correlated with affinity of a peptide to binding to a MHC class I molecule. Although in theory it would be possible to determine the affinity experimentally, in light of the huge number of combinatorial amino acid members it is more convenient to compute a theoretical pre-selected affinity value based on an algorithm well established and available to the skilled person (e.g. http://www.syfpeithi.de/home.htm; http://www.iedb.orgl).

- the combinatorial amino acid members in the data set are reduced to combinatorial amino acid members with a mass-to-charge ratio (m/z value) that is identical or its deviation does not exceed a given threshold value compared to a mass-to-charge ratio (m/z value) determined for a peptide or peptide fragment derived from an in vitro digest of the parent peptide, wherein the in vitro digest is performed using a proteasome, a 20S proteasome particle, prefereably of standard or immunoproteasome type, and/or immunoproteasome subtypes containing different ratios of the standard active site subunits β1, β2, β5 to β1i, β2i and β5i immunosubunits. Preferably the proteasome, the proteasome particle or one or more subunits thereof are of mammalian origin, more preferably they originate from rodent or human, in particular of mouse, rat or human origin. Preferably, the threshold value used during comparison of mass-to-charge ratios (m/z values) is set to allow an absolute deviation between two corresponding mass-to-charge ratios (m/z values) of not more than 0,5, preferably of not more than 0,2.

**Step d):**

In the method of the invention, for at least one peptide or peptide fragment present in or derived from a biological sample, the respective m/z value is determined. In order to be able to determine whether the at least one peptide or peptide fragment present in or derived from the biological sample represents a spliced peptide of the parent peptide, it is necessary to first determine the m/z value of said at least one peptide or peptide fragment. If its m/z value is available, said m/z value can be used for a comparison with the m/z values of the combinatorial amino acid members present in the data set. In this step, it is also possible to determine the m/z value of more than one peptide or peptide fragment present in or derived from the biological sample. Preferably the m/z value of the at least one peptide or peptide fragment is determined via mass spectrometry, more preferably by ionised mass spectrometry. Particular preferred ways of determining the m/z value of the at least one peptide or peptide fragment are ESI-MS, ESI-MS/MS, MALDI-MS, MALDI-MS/MS, LC-ESI/MS, LC-ESI-MS/MS, MALDI-TOF-MS, MALDI-TOF-MS/MS, MALDI-TOF/TOF-MS/MS, LC-MALDI-TOF/TOF-MS/MS, nano-LC-MALDI-TOF/TOF-MS/MS, ESI/MALDI LTQ Orbitrap.

Preferably, the at least one peptide or peptide fragment present in or derived from a biological sample, prior to determination of its mass-to-charge ratios (m/z values), has been processed by a proteasome, a 20S proteasome particle, preferably of standard or immunoproteasome type, and/or immunoproteasome subtypes containing different β1, β2, β5 standard subunit to β1i, β2i and β5i immunosubunit ratios. In other words, preferably the at least one peptide or peptide fragment has been exposed to proteasomal activity at least in one of above mentioned forms, at some point in time prior to determination of the m/z value of said processed at least one peptide or peptide fragment. Said processing of the at least one peptide or peptide fragment preferably happened under conditions that allow for proteasomal activity. Preferably, said processing of the at least one peptide or peptide fragment occurred in vitro (*i.e.* in solution in a test tube), ex vivo (i.e. in a cell or organ outside of an organism under conditions allowing for survival, activity or proliferation of said cell or isolated organ) and/or in vivo (i.e. in a cell or organ within an organism).

In the method of the invention, the at least one peptide or peptide fragment present in or derived from the biological sample, prior to determination of its mass-to-charge ratio (m/z value), has been isolated from a complex of said peptide or peptide fragment and a MHC class I molecule or a part thereof. Said complex may be formed between the at least one peptide or peptide fragment and a MHC class I molecule of any haplotype or any molecule comprising at least part thereof capable of binding a peptide and containing one or more peptide presenting $\alpha 1$ and $\alpha 2$ domains of an MHC class I molecule. Said complex may be presented on or derived from the surface of a living or dead cell or parts thereof. Said complex may also be the result of a purification or selection step that occurred during preparation or isolation of the at least one peptide or peptide fragment from its previous natural or artificial environment. For example, the at least one peptide or peptide fragment may have been isolated from a more complex biological sample by specific binding to and subsequent elution from immobilized MHC class I molecules or molecules comprising one or more peptide presenting $\alpha 1$ and $\alpha 2$ domains of an MHC class I molecule.

In the method of the invention it may be envisaged to pretreat the peptide or peptide fragment present in or derived from the biological sample prior to determining its m/z value. In particular, it may be taken care to remove some, most or all components from the peptide that may have been added to one or more of its amino acids e.g. during posttranslational modification (e.g. components added during glycosylation, isoprenylation or lipoylation).

**Steps e) and f):**

In a subsequent step of the method of the invention, the m/z value of the at least one peptide or peptide fragment present in or derived from the biological sample is compared to the m/z values of the combinatorial amino acid members comprised in the data set of step c). The skilled person is well aware of ways and methods of comparing m/z values. Various computer programs are available to the skilled person. If the m/z value determined for the at least one peptide or peptide fragment present in or derived from the biological sample and an m/z value of a combinatorial amino acid member of the data set is identical or its deviation does not exceed a given threshold value, the at least one peptide or peptide fragment of the biological sample is identified as spliced peptide of the parent peptide and optionally the amino acid sequence of the respective matching combinatorial amino acid member of the data set can be assigned to the at least one peptide or peptide fragment of the biological sample. Preferably, the threshold value used during comparison of m/z values is set to allow an absolute deviation between two corresponding m/z values of not more than 0,5, preferably of not more than 0,2. If two m/z values are found to differ by not more than 0,5, preferably by not more than 0,2, the two m/z values are considered as matching, as being identical and/or as referring to peptides with identical amino acid sequence.

In another aspect, the present invention is directed to a computer program, which, after it has been loaded into a memory appliance of a data processing device, enables said data processing device to conduct the method of the invention.

In a further aspect, the present invention is directed to a computer readable storage medium, on which a computer program is stored, which, after it has been loaded into a memory appliance of a data processing device, enables said data processing device to conduct the method of the invention.

The present invention is also directed to a method comprising downloading a computer program of the invention from an electronic data network, like e.g. the internet, onto a data processing device.

FIGURES:

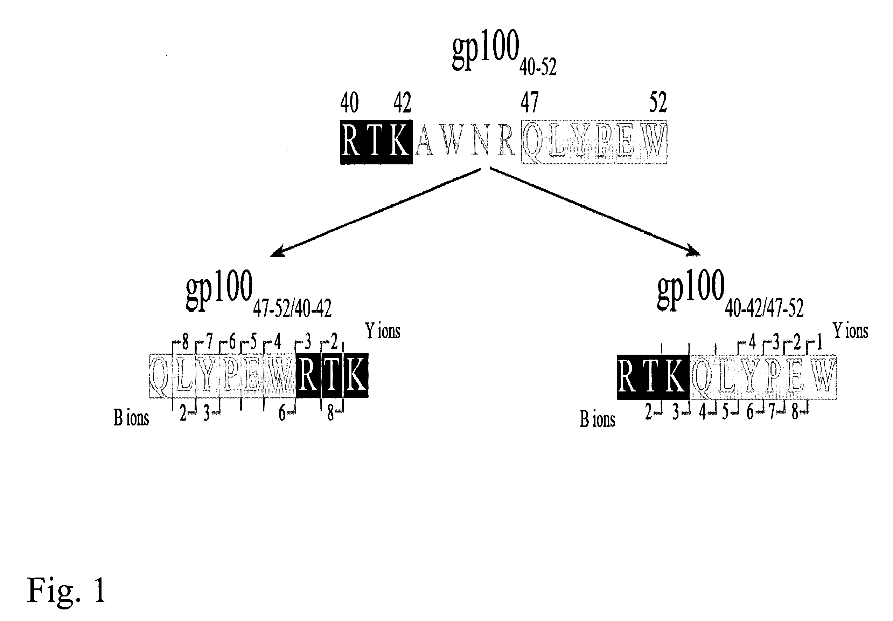

**Figure 1** Identification of PSP produced by digestion of the synthetic 13mer gp100$_{40-52}$ by 20S proteasomes. Sequences of parent peptide gp100$_{40-52}$ and spliced peptides are given as well as the B- and Y- ions identified during the MS/MS analysis.

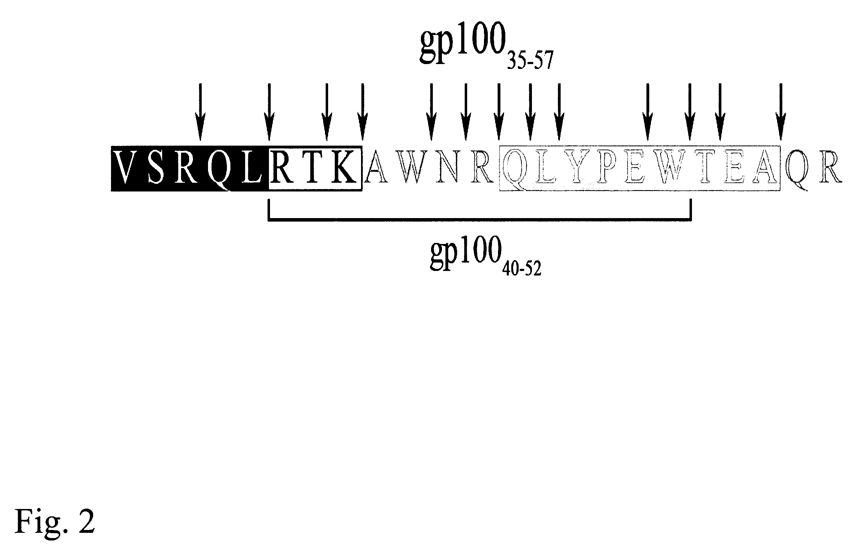

**Figure 2** Identification of PSP in gp100$_{35-57}$ digestion by SpliceMet. Sequence of gp100$_{35-57}$. The bracket indicates the previously described substrate gp100$_{40-52}$. Arrows indicate the cleavage positions that are necessary to generate the newly identified PSP.

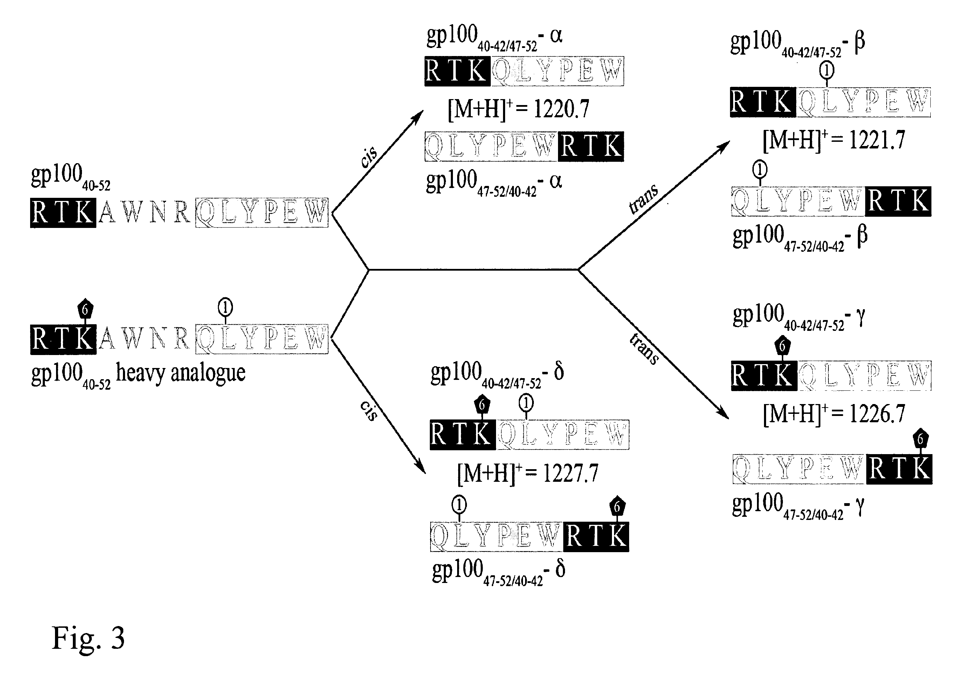

**Figure 3** Generation of PSP by *trans* splicing. To demonstrate the generation of a PSP$_{trans}$ by the binding of two fragments originated by two distinct molecules of substrate, 5 nmol gp100$_{40-52}$ and its heavy analogue with amino acids $^{15}$N-Leu and $^{13}$C$_6$-Lys (RTK$^{+6}$AWNRQL$^{+1}$YPEW) were digested together for 36 hours by 3 $\mu$g LcL 20S proteasomes in 100 $\mu$l buffer. Theoretically eight different PSP could be generated from the *cis* or *trans* ligation of the proteasomal fragments [RTK] and [QLYPEW] in normal or reversed order with sequences [RTK][QLYPEM] or [QLYPEM[RTK]: gp100$_{40-042/47-52}$-$\alpha$, gp100$_{47-52/40-42}$-$\alpha$, [M+H]$^+$ = 1220.7;

$gp100_{40-42/47-52}$-P. $gp100_{47-52/40-42}$-β, $[M^+H]^+$ = 1221.7; $gp100_{40-42/47-52}$-γ, $gp100_{47-52/40-42}$-γ. $[M+H]^+$ = 1226.7; $gp100_{40-42/47-52}$-δ, $gp100_{47-52/40-42}$-δ, $[M+H]^+$ = 1227.7.

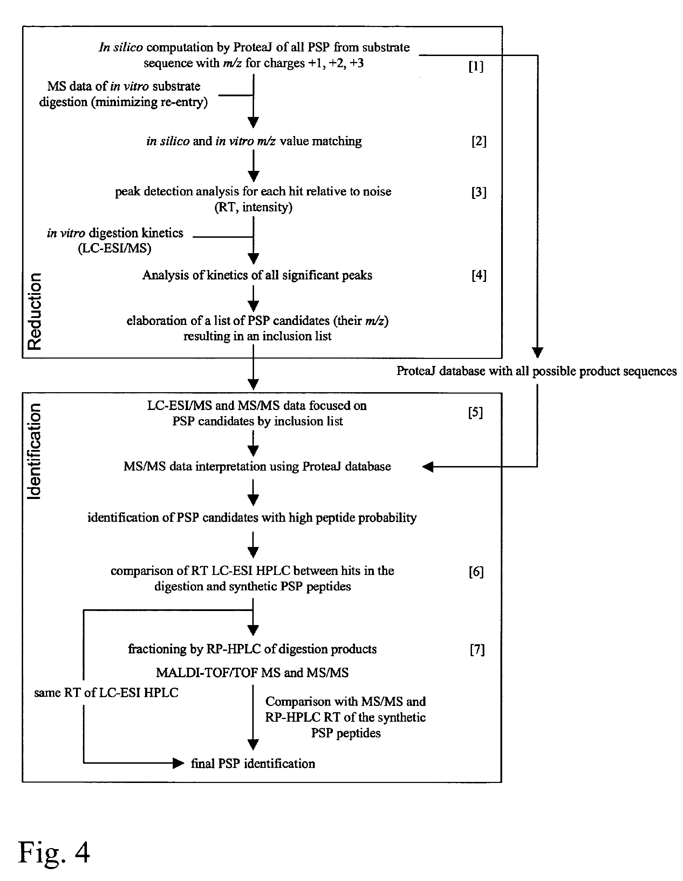

**Figure 4** SpliceMet. Applying the algorithm ProteaJ on a peptide sequence of choice, m/z values of all theoretically possible proteasomal cleavage (PCP) and splicing (PSP) products are calculated [1]. This is followed by an *in vitro* digest of the synthetic substrate and the comparison of the obtained MS signals with the theoretical m/z values [2]. Matching of the signals and verification of peptide generation kinetics results in an inclusion list for LC-ESI-MS/MS analysis required for identification of the PSP [3, 4]. For final confirmation the MS/MS spectra [5] and the HPLC-RT of proposed PSP [6] are compared with those of the analogous synthetic peptides. For the identification of those PSP candidates that do not fully satisfy these requisites or that need a further analysis because of their extreme novelty (e.g. $PSP_{trans}$), the generation of PSP is up-scaled followed by HPLC fractionation with an extended gradient and the fractions are analyzed by nano-LC-MALD)-TOF/TOF-MS [7].

EXAMPLES:

**SpliceMet**

To resolve the existing CTL-dependence of PSP identification an experimental approach was required that permitted a direct experimental and CTL independent identification of PSP. Therefore we here applied SpliceMet that combines the computer-based algorithm ProteaJ with proteasome *in vitro* digests of parent peptides of choice and mass spectrometry (MS) thereby permitting the direct and unrestricted identification of PSP. Based on a given parent peptide sequence, ProteaJ algorithm produces a data set with the m/z value of all theoretically possible combinatorial amino acid members (PSP) that may be generated by the proteasome through the combination of any two fragments (size > 1 amino acid) generated from the same substrate (*i.e.* in cis) or from separate substrates (*i.e.* in *trans*) and ligated in a normal or reverse order. This is followed by the MS analysis of *in vitro* digest of the synthetic parent peptide substrate and by comparison of obtained MS signals with the theoretical m/z values calculated by ProteaJ algorithm. By matching the theoretical with the experimentally obtained m/z values and verifying the peptide generation kinetics, a reduced data set of candidate PSP was generated. Their presence in 20S proteasome digests of the substrate was then investigated by LC-ESI-MS/MS and LC-MALDI-TOF/TOF-MS/MS leading to the final identification of the PSP.

**Validation of SpliceMet**

For proof of principle, we initially investigated 20S proteasome catalyzed peptide splicing during proteasomal degradation of the synthetic 13mer peptide ($gp100^{PMEL17}_{40-52}$, RTKAWNQLYPEW) previously shown to serve as substrate for PSP generation (Vigneron N, Stroobant V, Chapiro J, Ooms A, Degiovanni G, et al. (2004) An antigenic peptide produced by peptide splicing in the proteasome. Science 304: 587-590). For the experiments, we used 20S proteasomes of Lymphoblastoid cell Lines (LcL), which possess splicing activity and predominantly resemble the immunoproteasome subtype. Following SpliceMet, we identified two PSP by LC-ESI/MS/MS from the 5644 theoretically possible cis PSP (Fig. 1 and Table 2). Of these two, $gp100_{40-42/47-52}$ [RTK][QLYPEW] represented the previously reported spliced MHC class I epitope peptide (Vigneron N, Stroobant V, Chapiro J, Ooms A, Degiovanni G, et al. (2004) An antigenic peptide produced by peptide splicing in the proteasome. Science 304: 587-590).

**Identification of a PSP not generated by transpeptidation**

In contrast, with $gp100_{47-52/40-42}$ [QLYPEW][RTK] we identified a new PSP formed by ligation of the same peptides in a reverse order (Fig. 1). The production of both PSP could be blocked with lactacystin, demonstrating the proteasome dependence of the splicing reactions. Interestingly, LcL 20S proteasomes generated the PSP [QLYPEW][RTK] in a significantly higher amount than [RTK][QLYPEW] and with a relative high average efficiency of 4.54 ± 1.21 pmol per nmol of cleaved substrate. Both PSP were also generated by 20S proteasomes of T2 cells (standard proteasome) and T2.27 cells (immunoproteasomes) albeit with different efficiency when compared with LcL 20S proteasomes. Vigneron and coworkers proposed a transpeptidation mechanism for the generation of the spliced epitope peptide $gp100_{40-42/47-52}$ [RTK][QLYPEW] in which the N-terminus of the released peptide fragment, i.e. [QLYPEW] competes with the water molecules to make a nucleophilic attack on the ester bond of the acyl-enzyme intermediate thereby forming a new peptide bond producing the spliced peptides (Vigneron N, Stroobant V, Chapiro J, Ooms A, Degiovanni G, et al. (2004) An antigenic peptide produced by peptide splicing in the proteasome. Science 304: 587-590).

However, with the reversed PSP [QLYPEW][RTK], we identified a new spliced peptide which did not concur with the rules of a transpeptidation reaction and which had to be the result of a so far unprecedented reversed proteolysis reaction executed by the 20S proteasome.

**Identification of 12 new PSP in the digestion of gp100$_{35-57}$**

Although, gp100$_{40-42/47-52}$ and gp100$_{47-52/40-42}$ were readily identified when the gp100$_{40-52}$ peptide served as substrate, they could not be identified in LcL 20S proteasomes digests of the 23mer peptide gp100$_{35-57}$, which is a N- and C- terminally extended version of gp100$_{40-52}$ (Fig. 2). In comparison with the digests of the gp100$_{40-52}$ the relative amounts of the peptides [RTK] and [QLYPEW] generated per nmol of cleaved gp100$_{35-57}$ were strongly reduced, thereby suggesting that generation of the two PSP from gp100$_{35-57}$ was below detectable amounts. In addition, we identified two new PSP that were generated by the ligation of one of the two peptide fragments with other proteasomal cleavage products (PCP), *i.e.* gp100$_{47-55/40-42}$ [QLYPEWTEA][RTK] and gp100$_{47-52/35-37}$ [QLYPEW][VSR], indicating that [RTK] and [QLYPEW] peptides may compete with other PCP for the formation of different PSP. These are two examples of the twelve new PSP that we identified applying SpliceMet on the digestion of gp100$_{35-57}$ (Fig. 2 & Table 2). Intriguingly, for four of the new PSP we identified peptide bonds, which resembled ligations of two PCP necessarily derived from two separate substrate molecules. We called this phenomenon proteasomal *trans* splicing.

**Table 2 |** *PSP identified from substrates gp100$_{40-52}$ and gp100$_{35-57}$. The PSP identified by the application of SpliceMet on the proteasome-mediated digestion of the substrates gp100$_{40}$-$_{52}$ and gp100$_{35}$-$_{57}$ are here described. We demonstrated that PSP could be generated in vitro by cis or trans splicing. PSP$_{cis,normal}$ or PSP$_{cis,reverse}$ result from the splicing within one substrate molecule of substrate in the same or in reverse order as in the substrate, respectively; in contrast, PSP$_{trans}$ result from splicing of peptides derived from two distinct substrate molecules.*

| Peptide (gp100) | Sequence | Mr, calc | Type | Identification step of SpliceMet |
|---|---|---|---|---|
| **substrate gp100$_{40-52}$ - RTKAWNRQLYPEW** (SEQ ID No. 1) | | | | |
| 40-42/47-52 | [RTK](QLYPEW] (SEQ ID No. 2) | 1219.64 | PSP$_{cis,normal}$ | 6 |
| 47-52/40-42 | [QLYPEW][RTK] (SEQ ID No. 3) | 1219.64 | PSP$_{cis,reverse}$ | 6 |
| **substrate gp100$_{35-57}$ - VSRQLRTKAWNRQLYPEWTEAQR** (SEQ ID No. 16) | | | | |
| 47-55/35-39 | [QLYPEWTEA] [VSRQ L] (SEQ ID No. 4) | 1718.86 | PSP$_{cis,reverse}$ | 6 |
| 47-55/40-42 | [QLYPEWTEA] [RTK] (SEQ ID No. 5) | 1520.76 | PSP$_{cis,reverse}$ | 6 |
| 37-38/49-57 | [RQ] [YPEWTEAQR] (SEQ ID No. 6) | 1462.70 | PSP$_{cis,normal}$ | 7 |
| 45-52/35-37 | [NRQLYPEW] [VSR] (SEQ ID No. 7) | 1446.74 | PSP$_{cis,reverse}$ | 7 |
| 47-52/35-37 | [QLYPEW][VSR] (SEQ ID No. 8) | 1176.59 | PSP$_{cis,reverse}$ | 7 |
| 49-50/35-37 | [YPEW][VSR] (SEQ ID No. 9) | 935.45 | PSP$_{cis,reverse}$ | 6 |
| 49-52/35-39 | [YPEW][VSRQL] (SEQ ID No. 10) | 1176.59 | PSP$_{cis,reverse}$ | 6 |
| 47-48/35-39 | [QL][VSRQL] (SEQ ID No. 11) | 842.50 | PSP$_{cis,reverse}$ | 7 |
| 35-39/35-39 | [VSRQL][VSRQL] (SEQ ID No. 12) | 1184.70 | PSP$_{trans}$ | 6, 7 |

(continued)

| Peptide (gp100) | Sequence | Mr, calc | Type | Identification step of SpliceMet |
|---|---|---|---|---|
| 35-39/35-41 | [VSRQL] [VSRQLRT] (SEQ ID No. 13) | 1441.85 | $PSP_{trans}$ | 6, 7 |
| 47-53/53-57 | [QLYPEWT] [TEAQR] (SEQ ID No. 14) | 1520.73 | $PSP_{trans}$ | 6, 7 |
| 47-51/51-57 | [QLYPE] [EWTEAQR] (SEQ ID No. 15) | 1548.72 | $PSP_{trans}$ | 6, 7 |

### Cis and trans splicing

To verify the observation of an occurring *trans* splicing reaction we performed *in vitro* digests in which the unmodified 13mer gp100$_{40-52}$ peptide was applied to proteasomal processing in the presence of the same peptide but with the heavy amino acid residues $^{15}$N-Leu and $^{13}$C$_6$-Lys (RTK$^{+6}$AWNRQL$^{+1}$YPEW). As shown in Figure 3, we detected PSP variants being the results of cis (variants -α & -δ) or of *trans* (variants -β & -γ) splicing, demonstrating that proteasome-catalyzed splicing reactions indeed not only occurred in *cis* but also in *trans.* Furthermore, quantification of the cis and *trans* PSP variants suggested that *trans* splicing, at least *in vitro,* is not a rare event. Its existence implies the likely situation that two or more substrate molecules are present at the same time within the proteasomal cavity, or that the cleavage products of a first substrate molecule remain within the catalytic chamber while a second molecule of substrate is cleaved. Intriguingly, proteasomal *trans* splicing occurred both in a proposed transpeptidation- and in the reverse proteolysis-reaction since the *trans* PSP variants of both [QLYPEW][RTK] and [RTK][QLYPEW] were detected (Fig. 3).

### Quantifying the PSP generation

Comparison of the splicing activities of 20S proteasomes from T2 and T2.27 cells provided first insight into mechanistic aspects of proteasomal splicing and the correlation between the PSP generation and the amounts of involved PCP. The 23mer gp100$_{35-57}$ peptide was degraded considerably faster by T2.27 than by T2 20S proteasomes. Both proteasome isoforms produced the PSP [VSRQL][VSRQL] (gp100$_{35-39/35-39}$) and [QLYPEWTEA][VSRQL] (gp100$_{47-55/35-39}$) sharing the PCP [VSRQL], which was more efficiently generated by T2.27 than by T2 20S proteasomes. The enhanced liberation of [VSRQL] from the gp100$_{35-57}$ peptide by T2.27 proteasomes directly correlated with increased efficiency of PSP gp100$_{35-39/35-39}$ formation. In contrast, T2 and T2.27 20S proteasomes liberated the peptide [QLYPEW-TEA] (gp100$_{47-55}$) with similar efficiencies and there was no difference in the generation of the PSP gp100$_{47-55/35-39}$ indicating that in this case the limiting factor of the splicing reaction was the amount of gp100$_{47-55}$. This data suggested that while the two proteasome isoforms did not significantly differ in their peptide splicing capability, they did differ in their strength of cleavage site usage which consequently influences the amounts of generated proteasomal cleavage products and subsequently also the PSP formation. For example, T2.27 20S proteasomes, which produced higher amounts of PSP [VSRQL][VSRQL] and of PCP [VSRQL], possessed an average PSP/PCP ratio of $4.52 \pm 2.26$ pmol per nmol of released [VSRQL] peptide, which was similar to that of T2 20S proteasomes ($5.05 \pm 3.50$ pmol). Furthermore, when the PSP was produced by ligation of two different PCP, *i.e.* [QLYPEWTEA] and [VSRQL] the amount of only one of them exerted a correlation with the amount of generated PSP. This suggests that proteasome-catalyzed splicing is influenced by the affinity of the PCP for a given hypothesized macromolecular binding surface within the catalytic cavity of the 20S proteasome and hence by its retention that will allow peptide ligation.

### DISCUSSION

From our experiments, it emerged that 20S proteasomes possess a substantial *in vitro* peptide splicing activity. For example, T2 20S constitutive proteasomes completely degraded 4 nmol of the 23mer peptide substrate gp100$_{35-57}$ within 3 h thereby generating $52.5 \pm 29.3$ pmol of PSP gp100$_{47-55/35-39}$. That the amount of PSP generated per nmol of cleaved substrate did not significantly vary over time, even when the amount of substrate still present in the digestion strongly decreased also suggested that PSP formation was neither time dependent nor regulated by the concentration of the substrate.

Thus 20S proteasome catalyzed PSP epitope generation can reach efficiencies that are in the range of those previously obtained for non-spliced epitope peptides supporting the potential *in vivo* relevance of this activity. Furthermore, since *in vitro* experiments for generation of spliced and non-spliced epitope peptides are known to closely resemble the *in vivo* situation our data reveal that 20S proteasomes represent a molecular machine that is designed for the generation of spliced peptides from its own cleavage products. Overall our data have major biological implications in that they provide evidence that proteasome dependent protein degradation results in the generation of a second, so far undetected large pool of spliced peptides, from which novel functionally relevant peptides can be selected.

## METHODS

**I. SpliceMet.** The method SpliceMet can be organized in several main steps (see Fig 4). To reduce the number of combinatorial amino acid members (possible proteasome generated spliced peptides, PSP) various steps can be applied.

In a first embodiment, the method of the invention combines the computational algorithm ProteaJ with proteasome *in vitro* digests of a synthetic peptide of choice and mass spectrometric (MS) analyses. The stepwise reduction in the number of possible PSP for the peptides gp100$_{40-52}$ and gp100$_{35-57}$ by SpliceMet is described in Table 3.

**Table 3 |** *Reduction of number of PSP candidates during the progression of SpliceMet step by step. The number of possible PSP detectable in the in vitro digestion of a peptide declines continuously during the consecutive steps of SpliceMet. Here the PSP number reduction observed for the 13mer gp100$_{40-52}$ and 23mer gp100$_{35-57}$ is reported both as total number and as percentage compared to the theoretical PSP number (in brackets). The values are referring to the number of possible PSP at the end of the SpliceMet step. For example, although 5664 PSP could be generated from gp100$_{40-52}$ assuming 2 as the minimum length of the native PCP, only 2580 represent the mlz value clusters (obtained with a cluster range of 0.2) that will be matched with the LC-ESI/MS full spectrum at the beginning of step 2. Moreover, up to step 4 the numbers are referred to as the number of mlz values whereas from step 5 they are referred to as the possible sequence because they have been identified by MS/MS. In this table both cis and trans PSP are included.*

| SpliceMet steps | 1 | 2 | 3 | 4 | 5 | 6 | 7 |
|---|---|---|---|---|---|---|---|
| **gp100$_{40-52}$** | 2580 (100) | 280 (10.8) | 32 (1.2) | 18 (0.7) | 3 (0.1) | 2 (0.1) | |
| **gp100$_{35-57}$** | 7229 (100) | 1288 (17.8) | 1121 (15.5) | 239 (3.3) | 20 (0.3) | 4 (0.1) | 8 (0.1) |

The SpliceMet steps comprise:

1) *Calculation of all combinatorially possible PSP and setting of the ProteaJ database.* The digestion of the substrate of length L containing amino acids $a_i$, $i=1..L$ may result in $S_{cp} = \frac{1}{2}(L-L_{ext}+1)(L-L_{ext}+2)$ cleavage products (PCP) each of which can be denoted as $PCP_{ij}$, where the product starts at the position $i$, $i=1...L-L_{ext}+1$ (C-terminus) and ends at the position $j=i+L_{ext}-1...L$ (N-terminus). $L_{ext}$ describes the minimal length of a PCP that can produce a PSP. Any two $PCP_{ij}$ and $PCP_{kl}$ may be spliced into $PSP_{i-j/k-l}$. For the total amount of generated products $S_{all}$, including PCP and PSP, we have i=1... $L-L_{ext}+1$, j=i+$L_{ext}$-1...L, k=1...$L-L_{ext}$+1, l=k+$L_{ext}$-1...L and we can calculate

$$S_{all} = \sum_{i=1}^{L-L_{ext}+1} \sum_{j=i+L_{ext}-1}^{L} \sum_{k=1}^{L-L_{ext}+1} \sum_{l=k+L_{ext}-1}^{L} 1 .$$ Note that $S_{all}=\frac{1}{4}(L-L_{ext}+1)^2$ PSP can be classified into the two main groups, i.e. cis splicing (PSP$_{cis}$) and *trans* splicing (PSP$_{trans}$), whereby cis splicing occurs in the same order as in the substrate (PSP$_{cis,normal}$, where i+L$_{ext}$≤j+1≤k+1≤l-L$_{ext}$) or in reverse order (PSP$_{cis,reverse}$ k+L$_{ext}$≤l+1≤i≤j-L$_{ext}$+1).

The total number of all PSP is then $S_{PSP} = \frac{1}{4}(L - L_{ext} + 3)(L - L_{ext} + 2)(L - L_{ext} + 1)(L - L_{ext})$. The number of pure *trans* PSP can be calculated as PSP$_{trans}$=S$_{PSP}$-PSP$_{cis,normal}$- PSP$_{cis,reverse}$. Table 1 summarizes the conditions for each product and their total amount. *Estimation of mass-to-charge ratios (mlz) of all possible PSP.* To list all possible PSP, in which four indexes *ijkl* define the sequence, we computed the molecular weight (Mr, calc.) of each peptide and the corresponding $m/z$ values for charge states z = 1, 2, 3 ($m/z$ = (Mr+z)/z). Since the $m/z$ values of PSP can differ for less than the mass accuracy of 0.5 Da for the used ESI-ion trap mass spectrometer (LCQ-classic & DECA XP instruments), we clustered all $m/z$ values into groups with a m/z range smaller than 0.5 Da (0.2 Da in the present study). For each group we determined the average m/z value thereby obtaining a set of theoretical m/z values that could be further analyzed.

2) *Matching with the LC-ESI/MS full spectra.* The presence of the theoretical m/z values was detected among MS signals of the digestion products of the investigated peptide of choice.

3) *Peak detection of all the computed m/z values.* In the LC-ESI mass chromatogram we identified for each theoretical m/z value the significant peaks by peak detection algorithm with a signal-to-noise ratio larger than $\delta$ (here =2). For each theoretical m/z value either no or several peaks could be detected and defined by their m/z and retention time (RT).

4) *Analysis of m/z time-dependent kinetics and establishment of an inclusion list for the LC-ESI/MS measurements.* In time dependent processing experiments (signal intensity versus time of digestion) we analyzed the kinetics of the identified peaks by using LCQuan software version 2.5 (Thermo Fisher). Identified peaks that did not fulfill the following criteria were eliminated from the candidate list: i. initial intensity (t=0) smaller than MAX (e.g. here = $10^7$ for measurements by DECA XP MAX instrument); ii. monotonously ascending signal intensity towards a maximum followed by a monotonous decline in case assay condition allowed re-entry of the PSP. It was assumed that the monotonous increase resulted from the continuous production of PSP and the decrease from the "re-entry" event. Next, we defined $t_{max}$ as the digestion time when the highest amount of generated PSP was observed and sorted all pairs ($m/z$, RT) with respect to $t_{max}$ into groups indexed as g of the size $D_g$. If $D_g > D_{max}$ (here 15 depending on MS resolution) then the corresponding group was split into subgroups $g_i$ of the size smaller than $D_{max}$. The number of groups determined the number of additional up-scaled processing assays in which the absolute concentration of substrate and proteasome were increased keeping the relative substrate/proteasome ratio constant, whereas the total number of subgroups represented the number of requested new MS runs. The resulting (m/z, RT, $t_{max}$) established the inclusion list.

5) *LC-ESI-MS/MS analysis with inclusion list.* Precursor ion selection for MS/MS analysis was performed using the established inclusion list enabling the fragmentation analysis of even low abundant peptides. MS/MS spectra were analyzed with Bioworks software version 3.3 (Thermo Fisher) using the ProteaJ database. Significant hits which were annotated as PSP showed a peptide probability p < 0.00005.

6) *Comparison with synthetic peptides.* All identified PSP resulting from step 5 were manually confirmed by comparison with synthetic peptides of the same sequence. The candidate PSP and their synthetic analogues had to exhibit a similar RT ($\Delta$RT < 0.5 min) and fragmentation pattern in the LC-ESI-MS/MS analysis.

7) *Validation of PSP sequences by MALDI-TOF.* In some experiments the requirements outlined in step 5 and 6 were not fully met or, e.g. we detected *trans* PSP, requesting further MS identification. In this case, we proceeded by fractionating the digestion products by reverse phase (RP)-HPLC and by analyzing each fraction by LC-ESI-MS/MS using an inclusion list with the m/z values of the PSP candidates. Their RT in the HPLC run was also compared with that of the corresponding synthetic peptides. Those fractions with MS/MS and RT that matched the PSP were lyophilized and fractionated again using a more focused HPLC method to decrease the number of peptides in each fraction. The up-scaled fractions were subsequently compared with the RT of the synthetic PSP and analyzed by nano-LC-MALDI-TOF/TOF-MS/MS.

**II. Peptides and peptide synthesis.** The peptide sequences are reported in Table 2. All peptides were synthesized using Fmoc solid phase chemistry. The stepwise solid-phase synthesis of the peptide acids was performed on an automated ABI 433A peptide synthesizer from Applied Biosystems on a 0.1 mM scale, using conventional Fmoc/tBu strategy. As solid support preloaded TG-S-Trt resin (200-350 mg, loading 0.19-22 mmol/g) from Rapp Polymere GmbH or TCP resin from PepChem (150-250 mg, 0.51-0.54 mmol/g) was used. All amino acids were coupled according to standard methodologies in an automated single or double coupling mode. Fmoc deprotection was carried out with 20% piperidine in NMP. Exception had to be made for heavy analogue of gp100$_{40-52}$. The isotopic labelled amino acids $^{15}$N-Fmoc-L-Leucine (3eq. amino acid, 3eq. HBTU, 6eq. DIEA in DMF) and L-Lysine-N-Fmoc, N-T-Boc, $^{13}C_6$ (1.92eq. amino acid, 1.92eq. HBTU, 3.84eq. DIEA in DMF) were coupled over night. Cleavage of crude peptides from their resins was accomplished through treatment with 95% TFA 2.5% water, 2.5% TIPS for 1.5 or 3.5 h in case of Arg containing peptides. Preparative purification by high-pressure liquid chromatography (HPLC) was carried out on a Shimadzu LC-8A system with a Phenomenex-Gemini 10$\mu$, C18 (250 x 2.1 mm) column (Buffer A: 0.2% TFA in water, Buffer B: 0.2% TFA in water:acetonitrile, 1:4).

**III. Cell cultures.** Lymphoblastoid cell lines (LcLs) are human B lymphocytes immortalized with Epstein Barr virus (EBV) which mainly express immunoproteasomes. T2 cell line is a human T cell leukemia/B cell line hybrid defective in TAP1/TAP2 and LMP2/LMP7 subunits. T2.27 is a cell line originating from T2 cells and transfected with murine LMP2 and LMP7 subunits. LcLs and human T2 cell lines were cultured in RPM11640 medium supplemented with 10% FCS and, only for T2.27 cell lines, also with G418.

**IV. 20S proteasome purification.** 20S proteasomes were purified from 3*E+09 LcLs, T2 and T2.27 cells. Briefly, cells were homogenized in a 3-fold volume w/v of 20 mM Tris-HCl, 1 mM EDTA, 1 mM $NaN_3$, 1 mM DTT, pH 7.0 (TEAD buffer). The homogenate was centrifuged at 15000 g for 20 min and the supernatant subjected to ammonium sulphate fractionation. Proteins precipitating between a $(NH_4)_2SO_4$ saturation of 45-65% were dissolved in TEAD buffer and then dialyzed against the same buffer. The dialyzed protein solution was applied onto a column (5615 cm) of DEAE-Sephacel equilibrated with TEAD buffer. Bound proteins were eluted by a linear gradient of 0-500 mM NaCl dissolved in TEAD buffer. To detect the proteasome in the eluate, the collected fractions were tested for proteolytic activity with the fluorogenic substrate Suc-LLVY-MCA. Proteasome containing fractions were pooled and then subjected to gel filtration on a Sepharose 6B column (3680 cm) equilibrated in TEAD buffer. Fractions containing 20S proteasome were combined and further purified by chromatography on Mono Q (HR 5/5) in conjunction with a FPLC system (Amersham Biosciences, Freiburg, Germany). The proteasome was eluted from the column with an increasing NaCl/TEAD gradient (100-1000 mM). Then the proteasome containing fractions were pooled, $(NH_4)_2SO_4$ added to a final concentration of 1.2 M with respect to the salt and subjected to a Phenyl-Superose column (HR 5/5). From this column proteasomes were eluted with a linear decreasing gradient (1.2 - 0.0 M) of $(NH_4)_2SO_4$ dissolved in the TEAD and then extensively dialyzed against TEAD buffer. Purity of 20S proteasome preparation was verified by SDS-PAGE electrophoresis (12, 5 % poly-acrylamide gel stained with Coomassie dye). Furthermore, a non-proteasome proteolytic activity of the preparation was tested by the digestion of 40 $\mu$M gp100$_{40-52}$ for 24 hours by 1 $\mu$g of LcL 20S proteasomes in presence of 400 $\mu$M Lactacystin (previously incubated with 20S proteasomes at room temperature for 30 min). As reported, no production of PSP occurred.

**V. *In vitro* digestion of synthetic peptide substrates.** Synthetic peptides at different concentration (from 40 to 100 $\mu$M)were digested by 0.25-1.5 $\mu$g 20S proteasomes in 50-100 $\mu$l Hepes buffer (Hepes 20 mM, KCl 1 mM, MgCl 0.5 mM, DTT 1 mM, $NaN_3$ 1 mM, pH 7.3) for different time periods (from 20 min to 48 hours) at 37˚C. Digestions were stopped by acidic inactivation and frozen. Digestions were performed also in TEAD buffer (Tris 20 mM, EDTA 1 mM, $NaN_3$ 1 mM, DTT 1 mM, pH 7.2) and no remarkable differences compared to Hepes buffer emerged (data not shown). In contrast, for SpliceMet step 7, 1.1 $\mu$mol of the peptide gp100$_{35-57}$ (at the final concentration of 100 $\mu$M) were digested for 24 hours by 62 $\mu$g of LcL 20S proteasomes in 10 ml Hepes buffer and the products up-scaled by RP-HPLC separation. To compare the generation efficiency of the epitopes gp100$_{40-42/47-52}$ (PSP) and MBP$_{111-119}$ (PCP), 4 nmol gp100$_{40-52}$ and 7.5 nmol MBP$_{102-129}$ were digested in 100 $\mu$l TEAD buffer for different time by 1 $\mu$g and 0.4 $\mu$g LcL 20S proteasomes, respectively. All experiments reported in this study were repeated at least twice and each experiment set was measured by each MS instrument at least twice.

**VI. LC-ESI MS.** LC-runs were performed at different flow rates using 2 different gradients: 5-95 % solution B with an increase of the 6 % /min and 3-67% solution B with an increase of the 2 % /min. Peptide separation was carried out on a 2.1 mm ($\mu$RPC C2/C18, 100 mm x 2.1 mm, 3 $\mu$m, 120 Å, Amersham) and a 1 mm RP column (Beta Basic-18, 100 mm x 1 mm, 3 $\mu$m, 150 Å, ThermoFisher) using a Surveyor system (ThermoFisher Scientific, USA). Mobile phase (A) was 100% (v/v) water containing 0.05% (v/v) TFA and (B) was 70:30 (v/v) acetonitrile/water containing 0.045% (v/v) TFA or 0.1 % acetic acid for the PSP identifications reported. Online MS analysis was performed by DECA XP MAX iontrap instrument (ThermoFisher Scientific, USA) and by LCQ-classic iontrap (ThermoFisher Scientific, USA) after HPLC separation (HP1100, Agilent). MS data were acquired with a triple scan method in positive ion mode (MS - mass range 250 -2000 m/z, zoom scan, MS/MS). Analysis of ESI MS data was accomplished using Bioworks version 3.3 (ThermoFisher Scientific, USA). Database search was performed using ProteaJ database and the following parameters: no enzyme, mass tolerance for fragment ions 1amu.

**VII. Quantification of the PCP and PSP.** In order to obtain an estimation of the real amount of PSP and PCP from which the PSP had been generated, we combined two methods of peptide quantification, *i.e.* the titration by synthetic peptides of substrates, PSP and correlated PCP and an evolution of the Mass Balance Method (MBM), which estimates the amount of each PCP detected by MS through its MS signal. We performed the titrations of the substrates gp100$_{40-52}$ and gp100$_{35-57}$ (titration range: 0 - 40 $\mu$M), of the PCP RTK, QLYPEW and VSRQL (titration range: 0 - 10 $\mu$M) and of the identified PSP (titration range: 0 - 10 $\mu$M) in presence of 2 $\mu$g/ml inactivated 20S proteasomes. Because the biological-matrix (20S proteasome & peptides) could influence the MS signal detected for a specific peptide as was observed in our experimental setting through the signal quantification of the internal standard (YPHFMPTNLGPS, so called 9-GPS), we performed all the titrations by mixing substrates, PCP, PSP and inactivated 20S proteasomes in "realistic" concentrations. We titrated 13 peptides (with a length that varied between 6 and 23 amino acid) and calculated their conversion factors (MS signal / pmol of the peptide in the mix solution). The means of the conversion factors measured by DECA XP MAX or LCQ-classic instrument were 3.54*E+06 (+/-1.26*E+06) and 2.45*E+05 (+/-2,14*E+05), respectively. In MBM algorithm a pivotal parameter is the ratio Max/Min (of the conversion factors). We calculated this parameter for DECA XP MAX or LCQ-classic instruments as following: (mean + s.d.)/(mean - s.d.); means and standard deviation (s.d.) of the conversion factors have been computed from eight measurements by each MS instrument of mixes with different synthetic peptide concentrations. We obtained a ratio Max/Min of 2.11 for DECA XP MAX and 7.47 for LCQ-classic measurements. These values were adopted in the next analyses by MBM of the $gp100_{40-52}$ and $gp100_{35-57}$ digestions. Because the titration of the peptides shorter than 6mers showed a substantial difference in the conversion factor signal/pmol (data not shown) only the conversion factor computed by peptide titration were used for computing amount (pmol) of these PCP. For each digestion we also established the Substrate Cleavage Strengths (SCS) with the algorithm previously described. SCS describes the frequency on which proteasome cuts after each amino acid of the substrate sequence.

In order to investigate the efficiency of the production of PSP and PCP of 20S proteasomes we reported the pmol of PSP per nmol of substrate digested at a given time point. Therefore, if after 60 min we measured 200 pmol of PSP produced and 2 nmol of substrate cleaved in 100 $\mu$l of reaction, we reported the efficiency of production of that PSP as: 200 pmol (PSP) / 2 nmol (substrate consumed), that is 100 pmol/nmol. Through such a type of analysis we were able to compare digestions performed for different time (e.g. time-dependent kinetics) as well as digestions performed by 20S proteasomes with different degradation rate. Following the same approach we also investigated the correlation between a defined PSP and one of its native PCP by the formula: pmol (PSP) / nmol (PCP). Such a value provided an estimation of the pmol of PSP generated per nmol of correlated PCP released by 20S proteasomes. Because the degradation rate of the 23mer $gp100_{35-57}$ differed between T2 and T2.27 20S proteasomes, we compared the values pmol (PSP) /nmol (substrate consumed) and pmol (PSP) / nmol (correlated PCP) at: i. 40 min for T2.27 and 90 min for T2.27 20S proteasomes digestions (around 30% of substrate consumed); ii. 90 min for T2.27 and 180 min for T2 20S proteasome digestions (around 70% of substrate consumed); iii. 120 min for T2.27 and 240 min for T2 20S proteasome digestions (around 85% of substrate consumed); iv. the average for all time points. The differences between T2 and T2.27 20S proteasomes were substantially conserved from i. to iv. When we compared the generation efficiency of the epitopes $gp100_{40-42/47-52}$ (PSP) and $MBP_{111-119}$ (PCP) we reported the pmol (epitope) / nmol (substrate consumed) measured at different when the 40-60% of the substrate was already consumed.

To estimate the ratio between cis and *trans* PSP in the experiment reported, the peak areas of $gp100_{47-52/40-42}$ and $gp100_{40-42/47/52}$ at $[M+H]^+$ = 1220.7, 1221.7, 1226.7 and 1227.7 were calculated. Because isotopic peaks of the peptides with $[M+H]^+$ = 1220.7 and 1221.7 as well as 1226.7 and 1227.7 were overlapping in the MS spectra we deconvoluted the peaks on the basis of the isotopic pattern of the synthetic peptides $gp100_{47-52/40-42}$-$\alpha$ and $gp100_{40-42/47-52}$-$\alpha$ (first isotopic peak: 50.5 %, second: 33.2 %, third: 12.8% forth: 3.5 %). The sum of the peak areas of the variants -$\alpha$ & -$\beta$ represented the cis PSP and the variants -$\beta$ & -$\gamma$ the *trans* PSP. The sums of *cis* and *trans* PSP for both $gp100_{47-52/40-42}$ and $gp100_{40-42/47/52}$ were standardized and two experiments measured twice by LC-MALDI-TOF/TOF-MS were compared.

**VIII. Digestion product up-scaling by RP-HPLC.** Further identification of the PSP at step 7 of SpliceMet was performed by MALDI-TOF/TOF-MS analysis of the $gp100_{35-57}$ digestion products separated by two distinct rounds of RP-HPLC. In the first round 57 fractions were collected, lyophilized and analyzed by LC-ESI/MS to identify PSP candidates. The fractions containing the PSP candidates were then separated with more focused gradients (different for each selected fraction of the first round of HPLC separation) on the same column obtaining 47 fractions, which were lyophilized and investigated by MALDt-TOF/TOF-MS analysis. Each round was obtained by collecting the eluted fractions of the 5-15 runs (5-20 $\mu$l each) to maintain a good separation of the digestion products on the chromatogram. The runs were carried out on the column C18 (33 x 4.6 mm; ODS1 1.5 $\mu$m) by the HPLC Beckman SytemGold and different gradient of acetonitrile.

**IX. Nano-LC-MALDI-TOF/TOF-MS.** Peptide separation was carried out using an Ultimate HPLC system (Dionex, Idstein, Germany). Samples were concentrated on a trap column (PepMap C18, 5 mm x 300 $\mu$m x 5 $\mu$m, 100 Å, Dionex) and eluted onto an analytical column (PepMap C18, 150 mm x 75 $\mu$m x 3 $\mu$m, 100 Å, Dionex). Mobile phase (A) was 2:98 (v/v) acetonitrile/water containing 0.05% (v/v) TFA and (B) was 80:20 (v/v) acetonitrile/water containing 0.045% (v/v) TFA. Runs were performed at a flow rate of 200 nL/min using a binary gradient 0-15% B in 4 min, 15-60% B in 45 min, 60-100% B in 5 min. Column effluent was mixed with MALDI matrix (5 mg/ml $\alpha$-cyano-4-hydroxy-cinnamic acid in 70: 30 (v/v) acetontrile/water containing 0.1 % (v/v) TFA, 1 $\mu$l/min) and spotted at ten second intervals on MALDI steel targets using a Probot fractionation device (Dionex). MS analysis was performed on a 4700 Proteomics Analyzer (Applied Biosystems, Framingham, MA, USA). MS data were acquired in positive ion mode in the mass range 800-4000 *m/z* by accumulation of 1200 laser shots per spot and processed with default calibration. MS/MS spectra were generated by 1keV collisions and accumulation of 2500 to 10000 laser shots. Analysis of MALDI MS data was accomplished using MASCOT version 2.1 (Matrixscince, London, UK). Database search was performed using ProteaJ database and the following parameters: no enzyme, mass tolerance for precursors, +/- 80 ppm and for MS/MS fragment ions, +/- 0.3 Da. Spectral images for manual validation were prepared with Data Explorer Software version 4.8 (Applied Biosystems).

**Claims**

1.  Method for identifying spliced peptides of a parent peptide present in a biological sample, comprising the steps:

    a) providing an amino acid sequence of a parent peptide;
    b) determining all combinatorial amino acid members that can be generated by combination of two arbitrary fragments derivable from the amino acid sequence of the parent peptide and which thereby represent a potential spliced peptide of the parent peptide, with the proviso that each combinatorial amino acid member comprises a sequence of more than one amino acid;
    c) preparing a data set comprising determined combinatorial amino acid members, wherein each combinatorial amino acid member of the data set is assigned to its respective mass-to-charge ratios (m/z values);
    d) determining a mass-to-charge ratios (m/z values) for at least one peptide or peptide fragment present in or derived from a biological sample;
    e) comparing the mass-to-charge ratios (m/z values) of the at least one peptide or peptide fragment with the mass-to-charge ratios (m/z values) comprised in the data set; and
    f) if the mass-to-charge ratio (m/z value) of the at least one peptide or peptide fragment and a mass-to-charge ratio (m/z value) of a combinatorial amino acid member of the data set is identical or its deviation does not exceed a given threshold value, identifying the at least one peptide or peptide fragment as spliced peptide of the parent peptide and optionally assigning to the at least one peptide or peptide fragment the corresponding amino acid sequence of the respective combinatorial amino acid member of the data set.

2.  Method of claim 1, wherein the total number of combinatorial amino acid members in the data set is reduced to less than the total number of all combinatorial amino acid members determined in step b).

3.  Method of anyone of claim 2, wherein the determined combinatorial amino acid members of the data set of step c) consist of combinatorial amino acid members which can be generated solely by combination of two arbitrary fragments derivable from one single molecule of the amino acid sequence of the parent peptide (cis configuration).

4.  Method of claim 2, wherein the determined combinatorial amino acid members of the data set of step c) consist of combinatorial amino acid members which can be generated solely by combination of two arbitrary fragments derivable from two distinct molecules of the amino acid sequence of the parent peptide (*trans* configuration).

5.  Method of anyone of claims 2 to 4, wherein the combinatorial amino acid members in the data set are reduced to combinatorial amino acid members with a mass-to-charge ratio that is identical or its deviation does not exceed a given threshold value compared to a mass-to-charge ratio (m/z value) determined for a peptide or peptide fragment derived from an in vitro digest of the parent peptide, wherein the in vitro digest is performed using a proteasome, a 20S proteasome particle and/or immunoproteasome subtypes containing different ratios of the standard active site subunits β1, β2, β5 to β1i, β2i and β5i immunosubunits.

6.  Method of anyone of claims 2 to 5, wherein the combinatorial amino acid members in the data set are reduced to combinatorial amino acid members with an amino acid sequence length of 5 to 15 amino acids, preferably of 7 to 12 amino acids, more preferably 8 to 10 amino acids.

7.  Method of anyone of claims 2 to 6, wherein the combinatorial amino acid members in the data set are reduced to combinatorial amino acid members with a preselected affinity to a MHC class I molecule.

8.  Method of anyone of claims 1 to 7, wherein the at least one peptide or peptide fragment derived from a biological sample, prior to determination of its mass-to-charge ratio (m/z value), has been processed by a proteasome, a 20S proteasome particle and/or immunoproteasome subtypes containing different β1, β2, β5 standard subunit to β1i, β2i and β5i immunosubunit ratios.

9.  Method of claim 8, wherein said processing of the at least one peptide or peptide fragment occurred in vitro, ex vivo and/or in vivo.

10. Method of anyone of claims 1 to 9, wherein the at least one peptide or peptide fragment, prior to determination of its mass-to-charge ratio (m/z value), has been isolated from a complex of said peptide or peptide fragment and a MHC class I molecule or a part thereof.

11. Method of anyone of claims 1 to 10, wherein the mass-to-charge ratio (m/z value) of an amino acid sequence of a determined combinatorial amino acid member is calculated based on its molecular weight and for charge states z = 1, 2 and/or 3.

12. Method of anyone of claims 1 to 11, wherein the mass-to-charge ratio (m/z value) for the at least one peptide or peptide fragment derived from a biological sample is determined by mass spectrometry, in particular by ionised MS, ESI-MS, ESI-MS/MS, MALDI-MS, MALDI-MS/MS, LC-ESI/MS, LC-ESI-MS/MS, MALDI-TOF-MS, MALDI-TOF-MS/MS, MALDI-TOF/TOF-MS/MS, LC-MALDI-TOF/TOF-MS/MS, nano-LC-MALDI-TOF/TOF-MS/MS, ESI/MALDI LTQ Orbitrap.

13. Method of anyone of claims 1 to 12, wherein the threshold value used during comparison of mass-to-charge ratios (m/z values) is set to allow an absolute deviation between two corresponding mass-to-charge ratios (m/z values) of not more than 0,5, preferably of not more than 0,2.

14. Computer program, which, after it has been loaded into a memory appliance of a data processing device, enables said data processing device to conduct a method according to claims 1 to 13.

15. Computer readable storage medium, on which a computer program is stored, which, after it has been loaded into a memory appliance of a data processing device, enables said data processing device to conduct a method according to claims 1 to 13.

16. Method comprising downloading a computer program according to claim 14 from an electronic data network, like e.g. the internet, onto a data processing device.

Fig. 1

$$gp100_{35\text{-}57}$$

VSRQL RTK AWNR QLYPEWTEA QR

$$gp100_{40\text{-}52}$$

Fig. 2

Fig. 3

Fig. 4

# EP 2 362 225 A1

**Europäisches Patentamt**
**European Patent Office**
**Office européen des brevets**

## EUROPEAN SEARCH REPORT

Application Number

EP 10 07 5087

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DALET ALEXANDRE ET AL: "Splicing of distant peptide fragments occurs in the proteasome by transpeptidation and produces the spliced antigenic peptide derived from fibroblast growth factor-5." JOURNAL OF IMMUNOLOGY (BALTIMORE, MD. : 1950) 15 MAR 2010 LNKD- PUBMED:20154207, vol. 184, no. 6, 12 February 2010 (2010-02-12), pages 3016-3024, XP002590785 ISSN: 1550-6606 * abstract; figure 1, 3, 5, 6, 7; materials and methods; * ----- | 1-16 | INV. G01N33/68 |
| Y | VOIGT ANTJE ET AL: "Generation of in silico predicted coxsackievirus B3-derived MHC class I epitopes by proteasomes" AMINO ACIDS (VIENNA), vol. 39, no. 1, 9 December 2009 (2009-12-09), pages 243-255, XP002590786 * abstract; introduction; table 1; figure 2, 3; materials and methods; * ----- | 1-16 | |
| A | VIGNERON NATHALIE ET AL: "An antigenic peptide produced by peptide splicing in the proteasome." SCIENCE (NEW YORK, N.Y.) 23 APR 2004 LNKD-PUBMED:15001714, vol. 304, no. 5670, 23 April 2004 (2004-04-23), pages 587-590, XP002590787 ISSN: 1095-9203 * the whole document * ----- -/-- | 1-16 | TECHNICAL FIELDS SEARCHED (IPC) G01N |

The present search report has been drawn up for all claims

| Place of search | Date of completion of the search | Examiner |
|---|---|---|
| Munich | 7 July 2010 | Pilch, Bartosz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

25

Europäisches
Patentamt
European
Patent Office
Office européen
des brevets

**EUROPEAN SEARCH REPORT**

Application Number

EP 10 07 5087

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WARREN EDUS H ET AL: "An antigen produced by splicing of noncontiguous peptides in the reverse order." SCIENCE (NEW YORK, N.Y.) 8 SEP 2006 LNKD-PUBMED:16960008, vol. 313, no. 5792, 8 September 2006 (2006-09-08), pages 1444-1447, XP002590788 ISSN: 1095-9203 * the whole document * ----- | 1-16 | |

TECHNICAL FIELDS
SEARCHED       (IPC)

The present search report has been drawn up for all claims

| Place of search | Date of completion of the search | Examiner |
|---|---|---|
| Munich | 7 July 2010 | Pilch, Bartosz |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SCHWARTZ AL ; CIECHANOVER A.** Targeting proteins for destruction by the ubiquitin system: implications for human pathobiology. *Annu Rev Pharmacol Toxicol,* 2009, vol. 49, 73-96
- **KLOETZEL PM.** Antigen processing by the proteasome. *Nat Rev Mol Cell Biol,* 2001, vol. 2, 179-187
- **VIGNERON N ; STROOBANT V ; CHAPIRO J ; OOMS A ; DEGIOVANNI G et al.** An antigenic peptide produced by peptide splicing in the proteasome. *Science,* 2004, vol. 304, 587-590
- **WARREN EH ; VIGNERON NJ ; GAVIN MA ; COULIE PG ; STROOBANT V et al.** An antigen produced by splicing of noncontiguous peptides in the reverse order. *Science,* 2006, vol. 313, 1444-1447

- **BORISSENKO L ; GROLL M.** Diversity of proteasomal missions: fine tuning of the immune response. *Biol Chem,* 2007, vol. 388, 947-955
- **NANDI D et al.** The ubiquitin-proteasome system. *J Biosci,* 2006, vol. 31 (1), 137-55
- **KLOSS A ; MEINERS S ; LUDWIG A ; DAHLMANN B.** Multiple cardiac proteasome subtypes differ in their susceptibility to proteasome inhibitors. *Cardiovasc Res,* 2009
- **MISHTO M ; SANTORO A ; BELLAVISTA E ; SESSIONS R ; TEXTORIS-TAUBE K et al.** A structural model of 20S immunoproteasomes: effect of LMP2 codon 60 polymorphism on expression, activity, intracellular localisation and insight into the regulatory mechanisms. *Biol Chem,* 2006, vol. 387, 417-429

SEQUENCE LISTING

<110> Charite - Universitätsmedizin Berlin

<120> Method for identification of proteasome generated spliced peptides

<130> P715309EP

<160> 16

<170> PatentIn version 3.3

<210> 1
<211> 13
<212> PRT
<213> artificial

<220>
<223> parent peptide

<400> 1

Arg Thr Lys Ala Trp Asn Arg Gln Leu Tyr Pro Glu Trp
1               5                   10


<210> 2
<211> 9
<212> PRT
<213> artificial

<220>
<223> spliced peptide

<400> 2

Arg Thr Lys Gln Leu Tyr Pro Glu Trp
1               5


<210> 3
<211> 9
<212> PRT
<213> artificial

<220>
<223> spliced peptide

<400> 3

Gln Leu Tyr Pro Glu Trp Arg Thr Lys
1               5


<210> 4
<211> 14
<212> PRT
<213> artificial

<220>
<223> spliced peptide

<400> 4

Gln Leu Tyr Pro Glu Trp Thr Glu Ala Val Ser Arg Gln Leu
1               5                   10

```
<210>   5
<211>   12
<212>   PRT
<213>   artificial

<220>
<223>   spliced peptide

<400>   5

Gln Leu Tyr Pro Glu Trp Thr Glu Ala Arg Thr Lys
1               5                   10


<210>   6
<211>   11
<212>   PRT
<213>   artificial

<220>
<223>   spliced peptide

<400>   6

Arg Gln Tyr Pro Glu Trp Thr Glu Ala Gln Arg
1               5                   10


<210>   7
<211>   11
<212>   PRT
<213>   artificial

<220>
<223>   spliced peptide

<400>   7

Asn Arg Gln Leu Tyr Pro Glu Trp Val Ser Arg
1               5                   10


<210>   8
<211>   9
<212>   PRT
<213>   artificial

<220>
<223>   spliced peptide

<400>   8

Gln Leu Tyr Pro Glu Trp Val Ser Arg
1               5


<210>   9
<211>   7
<212>   PRT
<213>   artificial

<220>
<223>   spliced peptide

<400>   9

Tyr Pro Glu Trp Val Ser Arg
```

1                    5

<210>    10
<211>    9
<212>    PRT
<213>    artificial

<220>
<223>    spliced peptide

<400>    10

Tyr Pro Glu Trp Val Ser Arg Gln Leu
1                    5


<210>    11
<211>    7
<212>    PRT
<213>    artificial

<220>
<223>    spliced peptide

<400>    11

Gln Leu Val Ser Arg Gln Leu
1                    5


<210>    12
<211>    10
<212>    PRT
<213>    artificial

<220>
<223>    spliced peptide

<400>    12

Val Ser Arg Gln Leu Val Ser Arg Gln Leu
1                    5                    10


<210>    13
<211>    12
<212>    PRT
<213>    artificial

<220>
<223>    spliced peptide

<400>    13

Val Ser Arg Gln Leu Val Ser Arg Gln Leu Arg Thr
1                    5                    10


<210>    14
<211>    12
<212>    PRT
<213>    artificial

<220>
<223>    spliced peptide

<400>    14

```
Gln Leu Tyr Pro Glu Trp Thr Thr Glu Ala Gln Arg
1               5               10
```

```
<210>  15
<211>  12
<212>  PRT
<213>  artificial

<220>
<223>  spliced peptide

<400>  15
```

```
Gln Leu Tyr Pro Glu Glu Trp Thr Glu Ala Gln Arg
1               5               10
```

```
<210>  16
<211>  23
<212>  PRT
<213>  artificial

<220>
<223>  parent peptide

<400>  16
```

```
Val Ser Arg Gln Leu Arg Thr Lys Ala Trp Asn Arg Gln Leu Tyr Pro
1               5               10                  15
```

```
Glu Trp Thr Glu Ala Gln Arg
            20
```